# EUROPÄISCHE PATENTSCHRIFT

(11) **EP 1 008 646 B1**
(45) Veröffentlichungstag und Bekanntmachung des Hinweises auf die Patenterteilung: **12.09.2001**
(21) Anmeldenummer: 98811137.3
(22) Anmeldetag: 13.11.1998
(51) Int. Cl.: C12M 1/26, G01N 1/24

(54) **Nährbodenbehälter mit integrierter Luftansaug- und Luftführungsgeometrie zwecks Luftkeimbestimmung**
Culture medium container with integrated air suction and circulation geometry for aerial germs
Recipient pour milieu de culture à geométrie d' aspiration et de circulation d'air intégrée pour la détermination de germes aériens

(43) Veröffentlichungstag der Anmeldung: 14.06.2000
(73) Patentinhaber: van den Wildenberg, Pierre, 6330 Cham-Hüneberg (CH)
(72) Erfinder: van den Wildenberg, Pierre, 6330 Cham-Hüneberg (CH)
(74) Vertreter: BOVARD AG - Patentanwälte

(56) Entgegenhaltungen:
- WO-A-96/31594
- GB-A- 1 441 576
- GB-A- 2 224 118
- US-A- 3 001 914
- US-A- 3 795 135
- US-A- 3 938 366

## Beschreibung

Die vorliegende Erfindung betrifft Nährbodenbehälter mit integrierter Luftansaug- und Luftführungsgeometrie, die für Luftkeimbestimmung geeignet sind.

Die Luftkeimbestimmung nach der Impaktions-Methode, bei der handelsübliche Petrischalen verwendet werden, stellt eine der häufigsten angewandten Methoden dar. Bei allen bisher bekannten Anwendungen wird die Nährbodenschale der Petrischale in eine speziell konstruierte Ansaugkammer der Luftansaugeinheit eingeführt. Diese Ansaugkammer hat Ansaugöffnungen (Löcher bzw. Schlitze), durch welche die Raumluft angesaugt wird. Bestimmt durch den Luftvolumenstrom und die Geometrie der Ansaugöffnungen werden die Partikel im Luftstrom so stark beschleunigt, dass die Luftpartikel auf die darunterliegende Oberfläche des Nährbodens in die Nährbodenschale aufprallen und dort aufgenommen werden.

Diese Methode hat einen systembedingten Ablauf mit hohem Zeitaufwand und komplizierter Handhabung.
Die Petrischale muss zuerst geöffnet werden. Der Deckel muss während der Messzeit so gelagert werden, dass keine Sterilitätsfehler erfolgen. Die Nährbodenschale der Petrischale muss ohne Sterilitätsfehler in die Ansaugkammer des Gerätes eingeführt werden. Nach der Messung erfolgt das Ganze in umgekehrter Reihenfolge.

Durch die unterschiedlichen Füllhöhen der Nährböden (abhängig vom Hersteller) muss der Abstand zwischen der Nährbodenoberfläche und der Lufteintrittsebene, der Ansaugkammer, korrigiert werden. Wenn dies nicht erfolgt, ist ein Messfehler möglich.

Die hier vorgestellte Erfindung bezweckt die Aufhebung aller dieser Nachteile.

Die Erfindung hat einen Nährbodenbehälter mit integrierter Luftansaug- und Luftführungsgeometrie gemäss der Definition im Anspruch 1 zum Gegenstand.

Der erfindungsgemässe Nährbodenbehälter, der für Luftkeimbestimmung nach der Impaktions-Methode bestimmt ist, stellt eine Weiterentwicklung der Petrischale dar.

Der Nährbodenbehälter besteht aus einer Schale oder Wanne und einem Deckel. Die beiden Teile bestehen vorzugsweise aus gespritztem Kunststoff, z.B. Polycarbonat. Die Schale oder Wanne kann vom Hersteller oder Anwender mit Nährboden ausgestattet werden.

Der Deckel ist ausgestattet mit Luftansaugöffnungen, Löchern oder Schlitzen, die vorzugsweise eine optimierte Ansauggeometrie aufweisen. In einer bevorzugten Ausführungsform befindet sich im Zentrum des Deckels eine konische Versenkung zwecks mechanischer Verbindung zur Schale.

Die Schale weist, vorteilhafterweise im Zentrum, eine Ausstülpung auf, welche mit mindestens einer oder mehreren Öffnungen für den Luftaustritt ausgestattet ist. Die Ausstülpung weist zusätzlich stellt auch ein nach unten gerichtetes Loch dar, in welches die genannten Öffnungen münden. Das Loch besitzt vorzugsweise eine konische Form, zur Aufnahme eines Luftansauggerätes oder Luftftansaugschlauches. Im oberen Bereich der Ausstülpung ist gegebenenfalls ein konischer Bereich für die mechanische Verbindung mit der konischen Versenkung des Deckels vorgesehen.

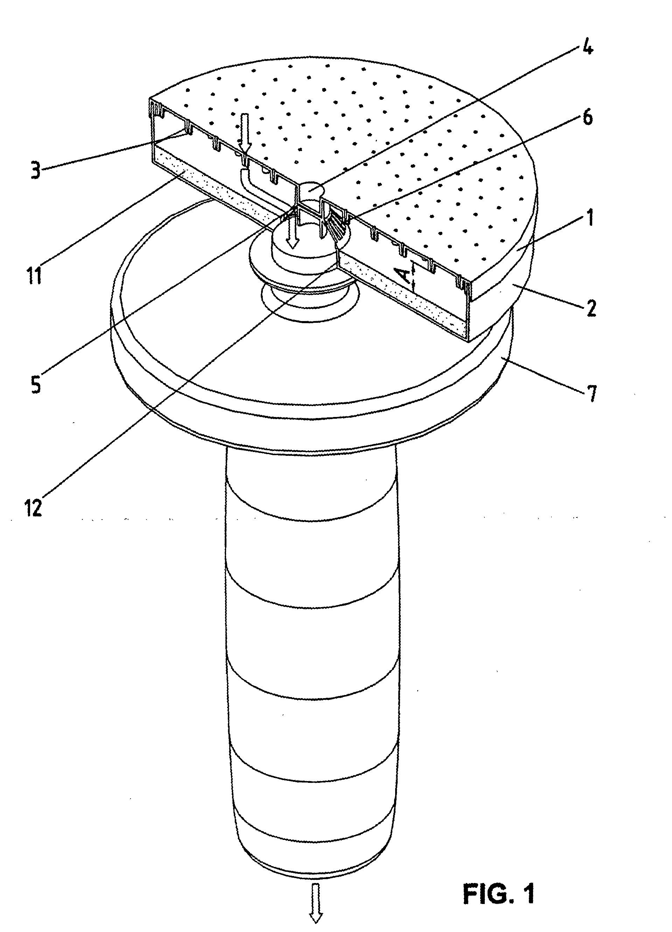

Die Nährbodenbehälter werden normalerweise steril abgefüllt und gestapelt geliefert. Ein Behälter kann zwecks Messung einfach entnommen werden und auf eine Luftansaugeinheit direkt oder über die Schlauchverbindung aufgesteckt werden. Danach erfolgt die Messung indem ein definiertes Luftvolumen in einer bestimmten Zeit angesaugt wird, z.B. zwischen 50 l bis zu 1000 l. Die Strömungssrichtung der Luft ist in Figur 1, Figur 3 und Figur 4 mittels Pfeilen angezeigt. Nach der Messung wird der Behälter entfernt, transportfähig gestapelt und anschliessend inkubiert. Der Behälterdeckel wird erst für die bakteriologische Analyse nach der Inkubation entfernt.

Spezielle Ausführungsformen von Nährbodenbehältem für die Luftkeimbestimmung gemäss der vorliegenden Erfindung können zusätzlich zu der Erfindungsdefinition gemäss Anspruch 1 folgende Merkmale allein oder in beliebiger Kombination aufweisen:

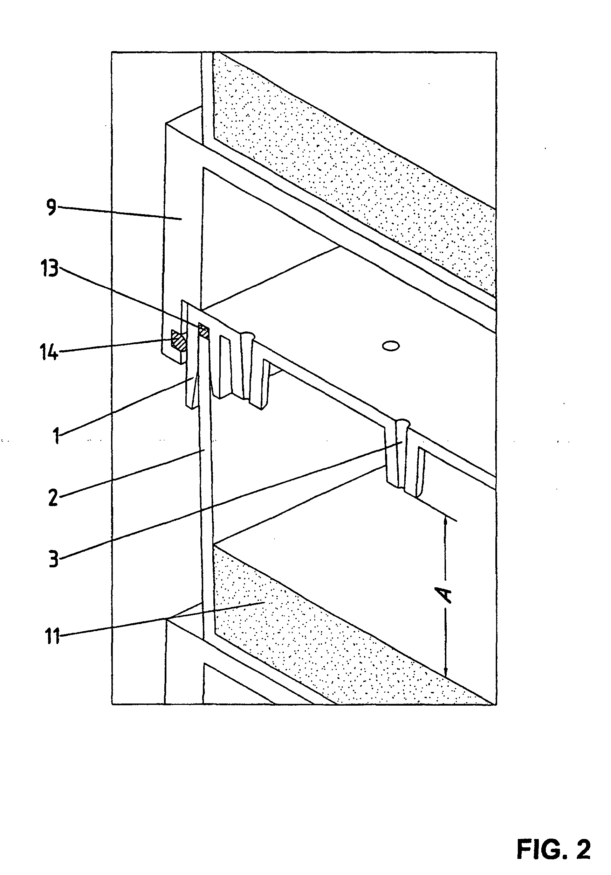

Eine systembedingt sehr eng tolerierte Impaktions-Distanz (Fig. 1, 2) zwischen der Lufteintrittsebene und der Nährbodenoberfläche.

Eine mechanisch stabile Verbindung zwischen dem Deckel und der Schale mittels Konussteckverbindung.

Eine luftdichte und hermetische Verbindung zwischen dem Deckel und der Schale, wenn nötig erfolgt die Abdichtung mittels O-Ring.

Der Deckel ist mit Luftansaugöffnungen ausgestattet.

Die Luftansaugöffnungen weisen eine optimierte Ansauggeometrie auf.

Die Luftansaugöffnungen können unterschiedliche Öffnungsgrössen aufweisen.

Es sollen verschiedene Deckel mit unterschiedlichen Öffnungsgrössen erhältlich sein, damit eine Differenzierung nach den Partikelgrössen möglich wird.

Anpassung der Nährbodenbehälter für eine kaskadenartige Anordnung von mehreren Nährbodenbehältern. Hierzu wird eine Kaskadenhaube vorgeschlagen, die mittels O-Ring eine hermetische Serieanordnung von z.B. drei Nährbodenbehältern übereinander, mit je einem Deckel unterschiedlicher Ansaugöffnungsgrössen, ermöglicht. Dies erlaubt eine Differenzierung nach Partikelgrössen.

Alle Systemteile weisen eine mechanische, stabile, konische Steckverbindung zueinander auf.

Der Nährbodenbehälterdeckel ist mit einer zentralen Versenkung versehen, die eine stabile Konusverbindung mit der Schale oder Nährbodenschale ermöglicht.

Die Nährbodenschale ist mit einer zentralen Ausstülpung versehen, zwecks stabiler Konusverbindung mit dem Nährbodenbehälterdeckel.

Die zentrale Ausstülpung der Nährbodenschale weist Luftdurchlassöffnungen auf.

Die Geometrie der Versenkung des Deckels und die Geometrie der Ausstülpung der Schale erlauben die Aufnahme eines Verbindungszapfens zwecks Stapelung.

Die Dimension des Verbindungszapfens kann so sein, dass bei Stapelung zwischen der Schale und dem Deckel zwecks Luftaustausch während der Inkubation ein Luftspalt bestehen bleibt.

Der Verbindungszapfen kann so ausgestaltet sein, dass ein Verschluss zwischen der Schale und dem Deckel erfolgt und so kein Spalt und kein Luftaustausch möglich ist.

Die Ausstülpung der Schale erlaubt eine mechanische stabile Konusverbindung zu Luftansaugschläuchen, Luftansaugeinheiten, Kaskadenhaube sowie zu dem Verschlusszapfen auf.

Der Nährbodenbehälter kann auf einer Luftansaugeinheit bzw. einem Luftabsaugschlauch durch eine einfache Steckverbindung plaziert werden.

Die vorliegende Erfindung wird beispielsweise mittels der beiliegenden Figuren 1 bis 5 näher erläutert.

Es zeigt Figur 1 eine perspektivische Darstellung eines Nährbodenbehälters gemäss der vorliegenden Erfindung im Schnitt, wobei der Behälter mit einer Luftabsauggerät verbunden ist.

Figur 2 zeigt eine perspektivische Teildarstellung eines Schnittes von kaskadenartig aufeinander gestapelten Nährbodenbehältem.

Figur 3 zeigt eine Teildarstellung eines Schnittes von kaskadenartig aufeinander gestapelten Nährbodenbehältern, wobei die Luftführungsgeometrie im Mittelpunkt steht.

Figur 4 zeigt eine perspektivische Darstellung eines Schnittes von kaskadenartig aufeinander gestapelten Nährbodenbehältem mit dazwischen liegenden Kaskadenhauben.

Figur 5 zeigt eine perspektivische Darstellung eines Stapelbehälters mit einem Schnitt entlang der zentralen Achse.

In Figur 1 ist eine perspektivische Darstellung eines Nährbodenbehälters gemäss der vorliegenden Erfindung dargestellt, zur besseren Erläuterung mit einem Schnitt durch die zentrale Achse. Der Nährbodenbehälter ist mit einem Luftansaugegerät 7 verbunden, welches für die Erzeugung eines Luftstromes durch den Nährbodenbehälter verantwortlich ist. Der Nährbodenbehälter besteht aus einem Deckel 1, einer Schale 2 und einem Nährboden 11. Zentral im Deckel befindet sich eine Versenkung 4, die eine konische Form besitzt, welche in ein Sackloch 5 der Ausstülpung 12 passt, die zentral im Innern der Schale 2 vorgesehen ist, wobei diese Verbindung für einen stabilen Sitz des Deckels 1 nützlich ist. Der Deckel 1 besitzt eine Vielzahl von regelmässig angeordneten Öffnungen 3, die in eine Düse münden, die in das Innere des Nährbodenbehälters gerichtet ist. Der Abstand der Düse der Öffnung 3 zum Nährboden ist konstant und weist den Wert A (Impaktions-Distanz) auf. Die in das Behälterinnere gerichtete Ausstülpung 12 der Schale besitzt Luftaustrittsschlitze 6, die regelmässig um die konische Form 5 angeordnet sind. Der Deckel 1 bildet mit der Schale 2 einen hermetischen Verschluss, wobei hierzu im Deckel eine Nut vorgesehen werden kann, in welcher sich ein O-Ring entsprechender Grösse befindet. Wenn das Ansauggerät 7 an die Ausstülpung der Schale angeschlossen ist, beginnt der Luftansaugvorgang. Dadurch wird im Schaleninnem ein Unterdruck erzeugt, wodurch Luft vom Äussern der Schale durch die Lufteintrittsöffnungen 3 ins Schaleninnere gesogen wird. Enthaltene Partikel werden auf den Nährboden "geschossen", wo sie haften bleiben. Zur Erzeugung des Unterdruckes im Schaleninneren wird die Luft durch die Luftaustrittsschlitze 6 ins Luftansauggerät 7 gesogen.

Figur 2 zeigt einen Ausschnitt einer Kaskadenanordnung von erfindungsgemässen Nährbodenbehältem. Zwischen der Schale und dem Deckel eines darunterliegenden Nährbodenbehälters ist eine Kaskadenhaube 9 angeordnet Die Kaskadenhaube 9 und der darunter liegende Deckel 1 bilden eine hermetisch abgeschlossene Kammer, wobei ein O-Ring 14 an der Kaskadenhaube für die Abdichtung vorgesehen ist Auf der Fig. 2 sind ebenfalls Einzelheiten des Deckels 1 vorgesehen, in der Nut am Rand des Deckels 1 befindet sich ein O-Ring 13, welcher für den hermetischen Verschluss mit der Schale 2 verantwortlich ist. Im Deckel sichtbar sind auch die Lufteintrittsöffnungen 3, welche als konische Düsen ausgebildet sind. Einfache Löcher im Deckel sind für den Zweck jedoch ebenfalls geeignet. Gut sichtbar ist auch die Impaktionsdistanz A zwischen dem Düsenende und dem Nährboden 11.

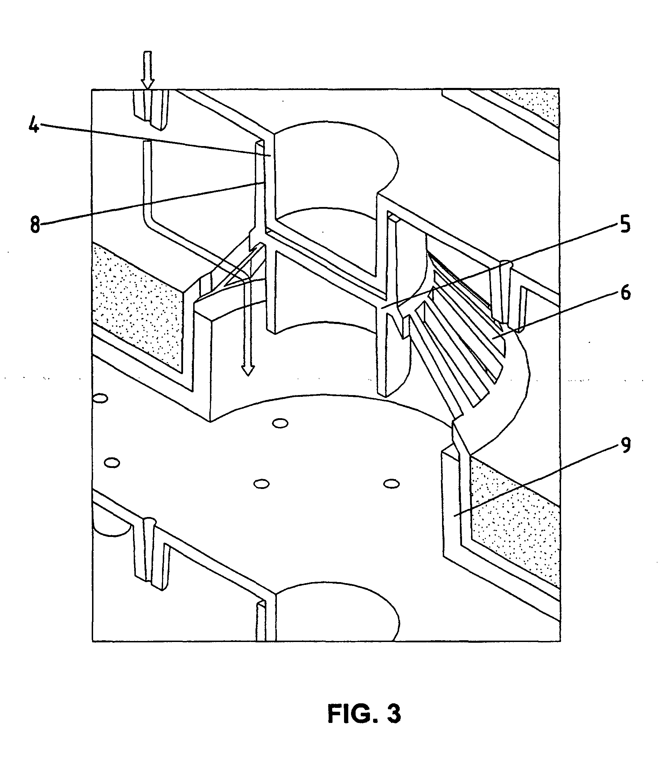

Figur 3 zeigt eine Teilansicht einer Kaskadenanordnung von zwei Nährbodenbehältern gemäss der vorliegenden Erfindung, mit einer dazwischen liegenden Kaskadenhaube 9. Die Kaskadenhaube 9 besitzt im Zentrum eine in einen Hohlzylinder auslaufende Öffnung, welche in die Ausstülpung der Schale 2 unter Ausbildung einer luftdichten Verbindung passt. Deutlich sichtbar ist die Versenkung 4 des Deckels 1, die mit der konischen Form 5 der Ausstülpung eine Konussteckverbindung 8 bildet. Deutlich sichtbar in dieser Teilansicht die Luftströmung, welche von der Öffnung des Deckels in den Nährbodenbehälter auf den Nährboden gelangt und von dort über die Austrittsöffnungen 6 in das Innere der Kaskadenhaube gelangt. Danach wird die Luft (in der Abbildung nicht dargestellt) in die Öffnungen des Deckels des darunter liegenden Nährbodenbehälters geführt. Eine solche Kaskadenanordnung kann beispielsweise drei Nährbodenbehälter umfassen, welche mit zwei Kaskadenhauben verbunden sind. Dabei ist es möglich, dass die Lufteintrittsöffnungen der Deckel 1 der verschiedenen Nährbodenbehälter verschieden gross sind. Diese Anforderung wird von gängigen Versuchsvorschriften gefordert.

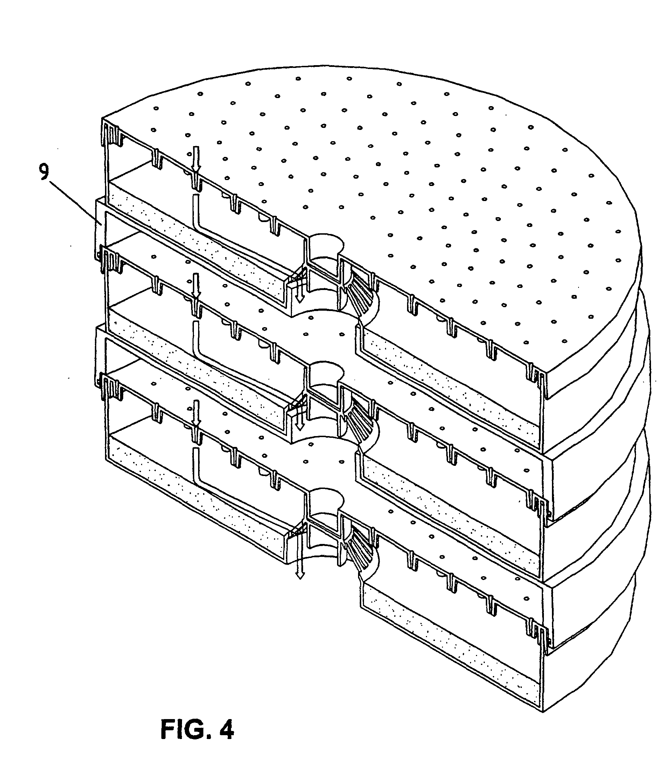

Figur 4 zeigt eine perspektivische Ansicht einer Kaskadenanordnung von 3 Nährbodenbehältem mit dazwischen liegenden Kaskadenhauben 9. Gut ersichtlich ist die durch Pfeile dargestellte Strömung der Luft.

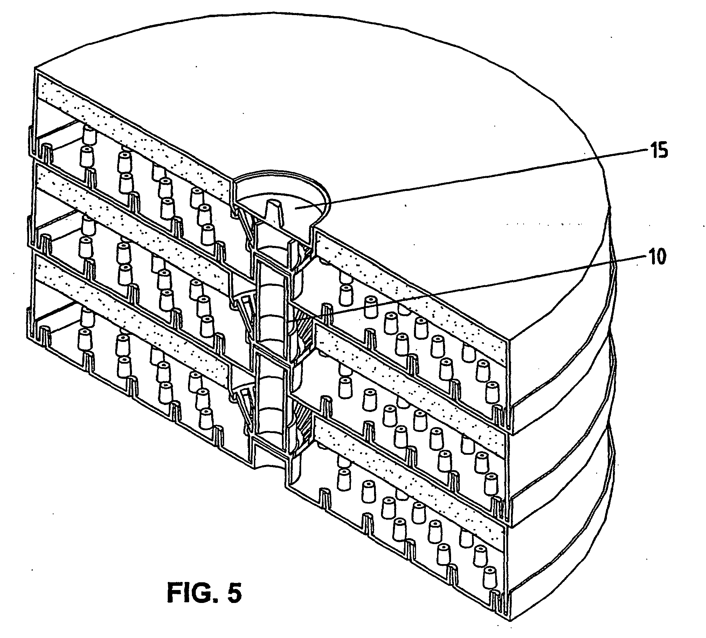

Figur 5 zeigt einen Stapel von drei erfindungsgemässen Nährbodenbehältern. Eine solche Anordnung ist beispielsweise für die Lieferung der Nährbodenbehälter mit bereits enthaltenem Nährboden nützlich. Ebenfalls kann diese Anordnung für die Inkubation nach dem Durchtritt der vorgeschriebenen Menge Luft verwendet werden. Eine Besonderheit dieses Stapels sind die Verbindungszapfen 10. Je nach der Länge dieser Zapfen kann zwischen den einzelnen Nährbodenbehältern ein grösserer oder kleinerer Luftspalt oder überhaupt kein Zwischenraum vorhanden sein. Zum hermetischen Abschluss von Nährbodenbehältern ist der Stopfen 15 vorgesehen. Dadurch wird die Kontamination des sterilen Innern der Nährbodenbehälter durch die Umgebungsluft verhindert. In einem solchen Fall müssen die Nährbodenbehälter ohne Zwischenraum gestapelt sein.

## Patentansprüche

1. Nährbodenbehälter, insbesondere für die Luftkeimbestimmung nach der Impaktions-Methode, bestehend aus einer Schale und einem passenden Deckel, **dadurch gekennzeichnet, dass** der Nährbodenbehälter eine integrierte Luftansaug- und Luftführungsgeometrie in Form von Öffnungen in Deckel und Schale aufweist.

2. Nährbodenbehälter nach Anspruch 1, **dadurch gekennzeichnet, dass** der Deckel in solcher Weise auf die Schale passt, dass ein hermetischer Verschluss gebildet wird, damit ein Lufteintritt allein durch die dafür vorgesehenen Öffnungen möglich ist.

3. Nährbodenbehälter nach Anspruch 1 oder 2, **dadurch gekennzeichnet, dass** für eine mechanisch stabile Verbindung zwischen dem Deckel und der Schale eine Konussteckverbindung vorgesehen ist

4. Nährbodenbehälter nach einem der Ansprüche 1 bis 3, **dadurch gekennzeichnet, dass** der Deckel mit Luftansaugöffnungen versehen ist und die vom Schalenboden nach oben gerichtete Ausstülpung mit Luftaustrittsöffnungen zentral in der Schale angeordnet ist.

5. Nährbodenbehälter nach Anspruch 4, **dadurch gekennzeichnet, dass** die zentrale Ausstülpung der Schale an ihrer oberen Seite eine konische Form mit einem konischen Sackloch aufweist und der entsprechende Deckel ein passenden konischen Zapfen aufweist, wobei Sackloch und Zapfen eine Konussteckverbindung bilden und die Luftaustrittssschlitze symmetrisch um die Konussteckverbindung herum angeordnet sind.

6. Nährbodenbehälter nach einem der Ansprüche 4 bis 5, **dadurch gekennzeichnet, dass** Deckel mit Luftansaugöffnungen von verschiedener Grösse vorgesehen sind, zwecks Differenzierung nach Partikelgrösse.

7. Nährbodenbehälter nach Anspruch 5, **dadurch gekennzeichnet, dass** die Konussteckverbindung in solcher Weise ausgebildet ist, dass bei Stapelung der Behälter je nach Zweck zwischen Schale und Deckel von aufeinanderfolgenden Gefässen ein Luftspalt vorhanden ist.

8. Nährbodenbehälter nach Anspruch 7, **dadurch gekennzeichnet, dass** die Grösse des Luftspaltes durch die Länge eines Zapfens gesteuert wird, der zentral zwischen Deckel und Schale eingesetzt wird.

9. Nährbodenbehälter nach einem der Ansprüche 1 bis 8, **dadurch gekennzeichnet, dass** er stapelbar ist.

10. Kaskadenhaube für Nährbodenbehälter nach einem der Ansprüche 1 bis 9, **dadurch gekennzeichnet, dass** sie passgenau und luftdicht abschliessend auf den Deckel des Nährbodenbehälters aufgesetzt werden kann, derart, dass zwischen dem Deckel und Kaskadenhaube ein Hohlraum entsteht; wobei die Kaskadenhaube auf ihrer Oberseite eine Öffnung aufweist, die in einen Hohlzylinder mündet, der unter Bildung einer luftdichten Verbindung in die Ausstülpung der Schale des Nährbodenbehälters passt.

11. Serielle Kaskadenanordnung von mindestens zwei Nährbodenbehältern nach einem der Ansprüche 1 bis 9 und mindestens einer Kaskadenhaube nach Anspruch 10.

12. Serielle Kaskadenanordnung nach Anspruch 11, **dadurch gekennzeichnet, dass** die Öffnungen in den Deckeln unterschiedliche Grössen aufweisen, damit eine Differenzierung der Partikelgrössen ermöglicht wird

## Claims

1. Culture medium container, in particular for air bacteria analysis according to the impaction method, comprising a dish and a fitted lid, **characterised in that** the culture medium container has an integrated geometry with respect to air suction and air conduction in the form of apertures in the lid and dish.

2. Culture medium container according to claim 1, **characterised in that** the lid fits on the dish in such a way that a hermetic seal is formed so that air admission is possible only through the apertures provided for that purpose.

3. Culture medium container according to claim 1 or 2, **characterised in that** a conical plug-in connection is provided for a mechanically stable connection between the lid and the dish.

4. Culture medium container according to one of the claims 1 to 3, **characterised in that** the lid is provided with air suction apertures, and the protuberance, directed upward from the dish bottom, and having air outlet apertures, is disposed centrally in the dish.

5. Culture medium container according to claim 4, **characterised in that** the central protuberance of the dish has on its upper side a conical shape with a conical blind hole, and the corresponding lid has a fitting conical peg, blind hole and peg forming a conical plug-in connection, and the air outlet slits being disposed symmetrically around the conical plug-in connection.

6. Culture medium container according to one of the claims 4 to 5, **characterised in that** lids are provided with air suction apertures of differing size for the purpose of differentiating according to particle size.

7. Culture medium container according to claim 5, **characterised in that** the conical plug connection is designed in such a way that upon stacking of the containers, depending upon the purpose, an air gap is present between dish and lid of successive receptacles.

8. Culture medium container according to claim 7, **characterised in that** the size of the air gap is controlled by the length of a peg which is inserted centrally between lid and dish.

9. Culture medium container according to one of the claims 1 to 8,
**characterised in that** it is stackable.

10. Cascade connecting hood for culture medium containers according to one of the claims 1 to 9, **characterised in that** it can be inserted, precisely fitted and closing in an airtight way, on the lid of the culture medium container in such a way that between the lid and the cascade connecting hood a hollow space arises, the cascade connecting hood having an opening on its upper side that comes out into a hollow cylinder, which cylinder fits into the protuberance of the dish of the culture medium container, while forming an airtight connection .

11. Serial cascade configuration of at least two culture medium containers according to one of the claims 1 to 9 and of at least one cascade connecting hood according to claim 10.

12. Serial case configuration according to claim 11, **characterised in that** the apertures in the lids have varying sizes so that a differentiation according to particle size is made possible.

## Revendications

1. Récipient pour milieu de culture, en particulier pour le dosage de germes de l'air selon la méthode de l'impact, constitué par une cuvette et un couvercle correspondant, **caractérisé en ce que** le récipient pour milieu de culture présente une géométrie intégrée d'aspiration et de cheminement de l'air sous la forme d'ouvertures dans le couvercle et la cuvette.

2. Récipient pour milieu de culture selon la première revendication, **caractérisé en ce que** le couvercle est adapté à la cuvette de telle manière qu'ils forment une fermeture hermétique afin que l'air ne puisse entrer que par les ouvertures prévues à cet effet.

3. Récipient pour milieu de culture selon la revendication 1 ou 2, **caractérisé en ce qu'**on obtient une fixation solide du couvercle sur la cuvette grâce à un raccord à emboîtement conique.

4. Récipient pour milieu de culture selon l'une des revendications 1 à 3, **caractérisé en ce que** le couvercle présente des ouvertures d'aspiration de l'air et que la saillie dirigée vers le haut depuis le fond de la cuvette, et qui présente des ouvertures de sortie de l'air, a une position centrale dans la cuvette.

5. Récipient pour milieu de culture selon la revendication 4, **caractérisé en ce que** la saillie centrale de la cuvette est, du côté supérieur, de forme conique avec un trou borgne conique et que le couvercle correspondant présente un téton conique adapté au trou, le trou borgne et le téton constituant un raccord à emboîtement conique et les fentes de sortie de l'air étant situées symétriquement autour de ce raccord.

6. Récipient pour milieu de culture selon la revendication 4 ou 5, **caractérisé en ce que** le couvercle présente des ouvertures d'aspiration de l'air de différentes tailles, et ce aux fins de différenciation en fonction de la grosseur des particules.

7. Récipient pour milieu de culture selon la revendication 5, **caractérisé en ce que** le raccord à emboîtement conique est réalisé de telle manière que, en cas d'empilement des récipients en fonction du but visé, il existe un interstice entre les cuvettes et les couvercles de récipients successifs.

8. Récipient pour milieu de culture selon la revendication 7, **caractérisé en ce que** l'importance de l'interstice est commandée par la longueur d'un téton qui est placé en position centrale, entre le couvercle et la cuvette.

9. Récipient pour milieu de culture selon l'une des revendications 1 à 8, **caractérisé en ce qu'**il peut être empilé avec des récipients identiques.

10. Chapeau pour montage en cascade destiné au récipient pour milieu de culture selon l'une des revendications 1 à 9, **caractérisé en ce qu'**il peut être placé, en s'adaptant exactement et en fermant d'une manière étanche à l'air, sur le couvercle du récipient pour milieu de culture, de telle manière qu'un espace vide soit formé entre le couvercle et le chapeau pour montage en cascade, ce chapeau présentant sur le dessus une ouverture qui débouche dans un cylindre creux, lequel s'adapte exactement, en formant une liaison étanche à l'air, dans la saillie de la cuvette du récipient pour milieu de culture.

11. Disposition en série et en cascade d'au moins deux récipients pour milieu de culture selon l'une des revendications 1 à 9 et d'au moins un chapeau pour montage en cascade selon la revendication 10.

12. Disposition en série et en cascade selon la revendication 11, **caractérisée en ce que** les ouvertures des couvercles sont de différentes tailles afin de permettre une différenciation entre les grosseurs de particules.
